Europäisches Patentamt

European Patent Office

Office européen des brevets

(19)

(11) **EP 0 851 762 B1**

(12) **EUROPÄISCHE PATENTSCHRIFT**

(45) Veröffentlichungstag und Bekanntmachung des Hinweises auf die Patenterteilung:
**17.04.2002 Patentblatt 2002/16**

(21) Anmeldenummer: **96932503.4**

(22) Anmeldetag: **12.09.1996**

(51) Int Cl.⁷: **A61K 38/18**
// (A61K38/18, 33:26),
(A61K38/18, 31:295,
A61P43:00)

(86) Internationale Anmeldenummer:
**PCT/EP96/03997**

(87) Internationale Veröffentlichungsnummer:
**WO 97/09996 (20.03.1997 Gazette 1997/13)**

(54) **PHARMAZEUTISCHE KOMBINATIONSPRÄPARATE ENTHALTEND ERYTHROPOIETIN UND EISENPRÄPARATE**

PHARMACEUTICAL COMBINATION PREPARATIONS CONTAINING ERYTHROPOIETIN AND IRON PREPARATIONS

PREPARATIONS PHARMACEUTIQUES COMBINEES CONTENANT DE L'ERYTHROPOIETINE ET DES PREPARATIONS A BASE DE FER

(84) Benannte Vertragsstaaten:
**AT BE CH DE DK ES FI FR GB GR IE IT LI LU NL PT SE**
Benannte Erstreckungsstaaten:
**LT LV SI**

(30) Priorität: **14.09.1995 DE 19535571**

(43) Veröffentlichungstag der Anmeldung:
**08.07.1998 Patentblatt 1998/28**

(73) Patentinhaber: **Roche Diagnostics GmbH**
**68298 Mannheim (DE)**

(72) Erfinder: **LEHMANN, Paul**
**D-67549 Worms (DE)**

(74) Vertreter: **Fouquet, Herbert, Dr.**
**F.Hoffmann-La Roche AG**
**Patentabteilung**
**Bau 675 / 3. OG**
**4070 Basel (CH)**

(56) Entgegenhaltungen:
**EP-A- 0 286 439**

• **JOURNAL OF THE AMERICAN SOCIETY OF NEPHROLOGY, 5 (3). 1994. 478., XP000616970 SUNDER-PLASSMANN G ET AL: "Optimizing low dose r-HuEPO combined with low dose I.V. iron therapy in hemodialysis patients"**

**Beschreibung**

Die vorliegende Erfindung betrifft pharmazeutische Kombinationspräparate enthaltend Eryhtropoietin und Eisenpräparate. Die Präparate werden insbesondere zur Behandlung von Anämie- oder Hämodialysepatienten eingesetzt.

Gegenstand der vorliegenden Erfindung ist ein pharmazeutisches Kombinationspräparat umfassend 2.000 - 7.000 U rekombinantes humanes Erythropoietin (rhEPO) und 1 - 20 mg einer äquivalenten Menge an Eisenionen eines physiologisch verträglichen Eisenpräparates, wobei das rhEPO und das Eisenpräparat in getrennten Darreichungsformen oder in einer einheitlichen Darreichungsform vorliegen können.

Es ist bekannt, die Anämie, insbesondere die transfusionsbedingte Anämie von Hämodialysepatienten, mit rekombinantem Erythropoietin zu therapieren. Die Anämie bei chronischen Krankheiten ist weltweit die zweithäufigste Anämieform.

Im Vordergrund der Anämien, die durch verminderte Erythropoese im Knochenmark oder durch Störungen in der Eisenreutilisation verursacht werden, steht eine verminderte Erythrozytenneuproduktion. Bei einem täglichen Rückgang der Erythrozytenneubildung um 1% kann die Anämie erst nach 1-3 Wochen klinisch festgestellt werden. Der tägliche Eisenbedarf für eine normale Erythropoese beträgt 25 mg. Davon stammt nur ca. 1 mg aus der Nahrung, der Hauptbedarf wird normalerweise durch Reutilisation des Hämoglobineisens nach dem Abbau gealterter Erythrozyten gedeckt. Bei chronischen Krankheiten ist die Eisenabgabe aus den Retikulumzellen stark vermindert. Das Eisen wird im retikuloendothelialen System festgehalten und steht nicht mehr für die Erythropoese zur Verfügung. Man spricht daher auch von einem 'inneren Eisenmangel', bei dem die Auslösung normaler Kompensationsmechanismen unvollständig erfolgt. Typisch sind eine Retikulozytopenie sowie das Fehlen einer zur Kompensation der Anämie erforderlichen Hyperplasie der Erythropoese. Eine verminderte Erythropoetinsekretion oder -aktivität kann ein zusätzlicher pathogenetischer Faktor sein. Eine signifikante Veränderung des Eisenstoffwechsels ist beispielsweise das Fehlen einer kompensatorisch vermehrten Transferrinbildung. Die Grundstörung liegt also in der fehlenden Abgabe des Eisens aus den Eisenspeichern (in den retikuloendothelialen Zellen) in das Plasma (somit auch in das Erythron), wodurch die normalen Kompensationsmechanismen nicht ausgelöst werden. Die Gabe von rekombinantem Erythropoetin wird therapeutisch genutzt, um einen signifikanten Anstieg der Erythrozytenzahl zu bewirken.

In der klinischen Chemie wird für die Diagnose der Anämie und von Eisenstoffwechselstörungen die Serumferritin-Konzentration bestimmt. Tritt zusätzlich zur Anämie der chronischen Krankheiten ein echter Eisenmangel auf, so steigt das Ferritin nicht an (meist bleibt es unter 90-95 ng/ml). Dieser Wert weist bei gleichzeitigen klinischen Zeichen von Infektion, Entzündung oder maligner Erkrankung auf eine Kombination von Eisenmangel und Anämie in Begleitung mit einer chronischen Erkrankung hin. Da das Serumferritin bei diesen Erkrankungen auch im Sinne eines Akute-Phase-Proteins reagieren kann, kann das Erythrozytenferritin besser diagnostisch verwertet werden.

Das Gesamtkörpereisen beträgt bei Männern ca. 3,5 g, bei Frauen 2,5 g. Eisen befindet sich im aktiven Metabolismus und in Speicherkompartimenten. Im aktiven Pool befinden sich bei einem Mann durchschnittlich 2100 mg im Hämoglobin, 200 mg im Myoglobin, 150 mg in Enzymen der Gewebe (Häm und Nicht-Häm) und 3 mg im Eisentransport-Kompartiment. Eisen wird im Gewebe intrazellulär als Ferritin (700 mg) und als Hämosiderin (300 mg) gespeichert.

Die Bioverfügbarkeit des Eisens kann pathopyhsiologisch gestört sein, so daß eine geringere Eisenabsorption im Körper erfolgt. Von den etwa 10 mg, die täglich mit der Nahrung verfügbar werden, resorbiert ein Erwachsener nur ca. 1 mg. Bei Eisenmangel steigt die Resorption, jedoch selten über 5-6 mg, wenn nicht zusätzliches Eisen zugeführt wird. Der genaue Resorptionsmechanismus für Eisen ist nicht geklärt. Die Regulation erfolgt in entscheidender Weise durch die Dünndarm-Mukosazellen. Das entscheidende Signal für die Mukosa scheint der Gesamteisengehalt des Körpers zu sein. Es wurde gezeigt, daß die Serum-Ferritinkonzentration invers korreliert mit der Menge des aufgenommenen Eisens.

Das Eisen wird von den intestinalen Mukosazellen an das Transferrin übergeben. Dieses Eisentransportprotein hat zwei Eisenbindungsstellen. Es wird in der Leber synthetisiert. Damit existiert ein Mechanismus, durch den Eisen von Zellen (z.B. Dünndarmmukosa, Makrophagen) übernommen und an spezifische Membranenrezeptoren von Erythroblasten, Plazentazellen oder Leberzellen abgegeben werden kann. Der Transferrin-Eisen-Rezeptor-Komplex gelangt durch Endozytose in die Erythrozyten-Vorläuferzellen, wo das Eisen an die Mitochondrien weitergegeben wird. Dort wird aus Eisen und Protoporphyrin Häm gebildet.

Nicht für die Erythropoese benötigtes Eisen wird mittels Transferrin in zwei Arten des Speicherpools verlagert. Der wichtigste Speicher ist das Ferritin. Hierbei handelt es sich um eine heterogene Familie von Proteinen, die einen Eisenkern umschließen. Es ist löslich und stellt die aktive Speicherform in Leber (Hepatozyten), Knochenmark, Milz (Makrophagen), Erythrozyten und im Serum (ca. 100 ng/ml) dar. Der Gewebeferritinpool ist sehr labil und rasch verfügbar, wenn Eisen benötigt wird. Das zirkulierende Serum-Ferritin stammt aus dem retikuloendothelialen System, und seine zirkulierende Konzentration geht parallel mit dem Gesamtkörpereisen (jedes ng/ml entspricht 8 mg Eisenvorrat).

Im Fall von Hämodialysepatienten hat sich gezeigt, daß der Eisenbedarf von mit rhEPO behandelten Patienten ganz erheblich ist. In der Regel wird bei diesen Patienten zusätzlich eine Eisentherapie durchgeführt, da das EPO nur dann seine optimale Wirkung entfalten kann, wenn die entsprechenden Eisenspeicher im Körper möglichst gefüllt sind. Um die Eisenspeicher möglichst aufzufüllen, war es bisher üblich, hohe Dosen von Eisenpräparate zu verabreichen. Allerdings können zu hohe Dosen an Eisenpräparaten auch zu unerwünschten Nebenwirkungen bei den Patienten führen. Insbesondere ist die intravenöse Applikation von Eisenpräparaten wegen der extremen Toxizität von Eisenionen physiologisch nicht unbedenklich. Von der Anwendung bestimmter Eisenpräparate bei Patienten mit bekannter allergischer Reaktionslage, z.B. bei Asthmatikern, wird in der Regel sogar abgeraten. Die Beurteilung des Füllzustandes der Eisenspeicher ist durch Bestimmung des Proteins Ferritin und durch Bestimmung der Transferrinsättigung möglich (M. Wick, W. Pingerra, P. Lehmann "Eisenstoffwechsel. Diagnose und Therapie der Anämien", Seiten 5-14, 38-55, 65-80, 94-98; Dritte erweiterte Auflage, September 1996. Springer Verlag Wien, New York), wobei die Transferrinsättigung den Eisenfluß von den Depots zum Knochenmark repräsentiert, während der Serum-Ferritin-Wert ein Maß für das gespeicherte Eisen ist.

Die Eisenspeicher gelten als "gefüllt", wenn das Serum-Ferritin > 150 $\mu$g/l ist und eine Transferrin-Sättigung > 20 % vorliegt. P. Grützmacher et al. beschreiben in Clinical Nephrology, Vol. 38, Nr. 1, 1992, S. 92-97, daß man unter diesen Bedingungen von einem maximalen Ansprechen auf die EPO-Therapie ausgehen kann.

Man spricht gegenwärtig bei der Eisentherapie von EPO-behandelten Dialysepatienten von einer "Korrekturphase" und einer "Erhaltungsphase". In der Korrekturphase werden möglichst hohe Dosierungen an Eisenpräparaten verabreicht, um die Eisenspeicher möglichst schnell aufzufüllen. Die Applikation von geeigneten Eisenpräparaten erfolgt dann zweckmäßigerweise als intravenöse Bolusinjektion. In der Erhaltungsphase werden die Eisenspeicher dann mit niedrigeren Eisendosen "gefüllt gehalten". Die Applikation von geeigneten Eisenpräparaten erfolgt in dieser Phase nicht mehr als schnelle Bolusinjektion, sondern in Form von üblichen Infusionspräparaten oder durch orale Gabe.

Der Eisen-Bedarf des rhEPO-behandelten Hämodialyse-Patienten kann sowohl in der Korrektur- als auch in der Erhaltungsphase ganz erheblich sein. Für die Synthese von 1 g/dl Hämoglobin in der Korrekturphase sind 150 mg Eisen erforderlich, die entweder aus endogenen Eisenspeichern gedeckt oder exogen zugeführt werden müssen. Auch in der Erhaltungsphase ist der Eisen-Bedarf gesteigert, da es bei Hämodialysepatienten bei jeder Behandlung zu kleineren Blutverlusten kommt. Über den Zeitraum eines Jahres schätzt man den Eisen-Verlust auf etwa 1000 mg Eisen (3 mg/Tag). Ein solcher Verlust kann langfristig nur exogen ausgeglichen werden. Hierzu stehen im Prinzip orale und intravenöse Darreichungsformen zur Verfügung.

Da die orale Eisenresorption nur ca. 1 mg/Tag, unter extremer Belastung (bei oraler Verabreichung von ca. 300 mg Fe(III)/Tag) weniger als 3 mg/Tag, beträgt, wird zunehmend die intravenöse Applikation größerer Eisenmengen bevorzugt. Auf dem deutschen Arzneimittelmarkt stehen momentan zwei intravenös applizierbare Eisen-Präparate zur Verfügung. Dabei handelt es sich um die Medikamente "Ferrlecit" und "Ferrum Vitis". Ferrlecit ist ein Eisen-(3)-Gluconat-Komplex. während "Ferrum Vitis" ein Eisen-(3)-Hydroxid-Saccharat-Komplex ist.

Die vielfältigen Probleme einer hochdosierten, langfristigen oralen Eisen-Therapie lassen sich zwar relativ einfach durch die intravenöse, subcutane Applikation von Eisen(III) während der Hämodialysebehandlung umgehen, da hierbei ein sicherer intravenöser, subcutaner Zugang besteht und die Injektion ohne weitere Belastung für den Patienten erfolgen kann. In den letzten Jahren hat dieses Verfahren zunehmend Verbreitung gefunden, da man davon ausging, daß mit den Präparaten "Ferrlecit" und "Ferrum Vitis" relativ nebenwirkungsarme Darreichungsformen zur Verfügung stehen. Inzwischen wurde aber auf Nebenwirkungen im Zusammenhang mit der Ferrlecit-Therapie bei autologer Bluttransfusion hingewiesen und die Indikation der parenteralen Ferrlecit-Therapie deutlich eingeengt. Es wird auf die Möglichkeiten von Kreislaufreaktionen bis hin zum Kollaps sowie auf das mögliche Auftreten von anaphylaktischen Reaktionen aufmerksam gemacht. Ferner wird eine höchstzulässige Tagesdosis von 2 Ampullen zu 5 ml entsprechend 125 mg Eisen festgelegt.

Die intravenöse Verabreichung beider Eisen-Präparate ist also nicht trivial, da bei der Applikation beider Medikamente mit Nebenwirkungen zu rechnen ist, vor allem dann, wenn größere Mengen relativ schnell injiziert werden müssen. Ferner kann die intravenöse Verabreichung der Eisenpräparate Probleme bereiten bis hin zu Akute-Phasen-Reaktionen, wenn die Eisendosis zu hoch bzw. nicht optimal abgestimmt mit der EPO-Dosis gegeben wird.

Offensichtlich sind die hohen Eisen-Dosierungen, die EPO-behandelten Dialysepatienten verabreicht werden müssen, nachteilig. Es steigt das Risiko eines Myokard-Infarktes und auch das Risiko der Entwicklung einer Eisencirrhose ist signifikant erhöht. Im Rahmen der Behandlung von Dialysepatienten ist eine adäquate Eisenzufuhr sowie eine geeignete Methode zur Bestimmung des Eisenmangels von erheblichem therapeutischen Nutzen, da nicht ausreichende Eisenverfügbarkeit eine der Hauptursachen für eine nicht ausreichende Wirkung von EPO bzw. für eine EPO-Resistenz ist.

Aufgrund einer zu hohen Dosierung von eisenhaltigen Präparaten kann es auch zu Eisenvergiftungen kommen. Elementares Eisen hat einen toxischen Effekt auf den Gastrointestinaltrakt, das kardiovaskuläre und des zentralnervöse System. Die oral letale Dosis elementaren Eisens schwankt zwischen 200 und 250 mg/kg. Die am häufigsten verwendeten Eisentabletten sind Ferrosulfat (enthält ca. 20 % elementares Eisen), Ferrofumarat (enthält etwa 30 % elementares Eisen) oder Ferroglukonat (enthält ca. 10 % elementares Eisen).

Es gibt vier typische Stadien der Eisenvergiftung: Stadium I (innerhalb der ersten 6 Stunden nach Vergiftung): Erbrechen, Diarrhö, Hyperirritabilität, Bauchschmerzen, Anfälle, Apathie und Koma können auftreten. Irritationen der gastrointestinalen Mukosa können zu einer hämorrhagischen Gastritis führen. Bei hohen Serumeisenspiegeln können Tachypnoe, Tachykardie, Hypotension, Schock, Koma und metabolische Azidose auftreten. Stadium II (innerhalb der ersten 10-14 Stunden nach Vergiftung): Während einer Latenzperiode, die bis zu 24 Stunden dauern kann, kommt es zu einer scheinbaren Verbesserung. Stadium III (12-48 Stunden nach Vergiftung): Schock, Hypoperfusion und Hypoglykämie treten auf. Serumeisenspiegel können normal sein. Leberschäden mit erhöhter GPT, Fieber, Leukozytose, Gerinnungsstörungen, T-Inversion im EKG, Orientierungsstörungen, Ruhelosigkeit, Apathie, Anfallsneigung, Koma, Schock, Azidose und Tod können auftreten. Stadium IV (2-5 Wochen später): Mögliche Komplikationen seitens einer Pylorus-, Antrum- oder einer anderen intestinalen Obstruktion, einer Leberzirrhose oder einer Schädigung des Zentralnervensystems können in den Vordergrund treten.

Aufgabe der Erfindung war es, ein Kombinationspräparat aus rekombinantem humanen Erythropoietin und einem Eisenpräparat bereitzustellen, das eine für die Therapie von Eisenstoffwechselstörungen optimal abgestimmte Menge von EPO und Eisenionen enthält. Insbesondere sollen mit Hilfe dieser Kombinationspräparate die gezeigten Risiken, vor allem die Akute-Phase-Reaktionen, vermieden werden. Bei Patienten, die mit rhEPO behandelt werden, soll ferner eine optimale EPO-Wirkung erzielt werden, sowie eine EPO-Resistenz vermieden werden.

Das erfindungsgemäße Kombinationspräparat umfaßt 2.000 - 7.000 U rhEPO und 1 - 20 mg einer äquivalenten Menge an Eisenionen eines physiologisch verträglichen Eisenpräparates, insbesondere eines Fe(II)- oder Fe(III)-komplexes. wobei das rhEPO und das Eisenpräparat als Kombinationspräparate vorliegen.

Im Sinne der vorliegenden Erfindung sollen unter dem Begriff "Kombinationspräparate" nicht nur solche Arzneimittelpackungen verstanden werden, bei denen das EPO und das Eisenpräparat in einer verkaufsfertigen Verpackungseinheit nebeneinander konfektioniert vorliegen (sogenannte Kombinationspackung), sondern auch solche Arzneimittelpackungen, die entweder eine geeignete Menge an EPO oder eine geeignete Menge eines Eisenpräparates in Form eines Einzelpräparates enthalten, wobei die Einzelpräparate hinsichtlich der Menge der Inhaltsstoffe derart konfektioniert sind. daß sie im Sinne der Erfindung für die kombinierte Gabe mit dem jeweils anderen Präparat verabreicht werden können. In diesen Fällen werden von den Pharma-Herstellern oder den Arzneimittel-Importeuren den Präparaten in der Regel ein in vielen Ländern gesetzlich vorgeschriebener Beipackzettel für Arzneimittel beigelegt, in dem Anweisungen oder Informationen über die kombinierte Gabe der Einzelpräparate enthalten sind. Die Kombinationspräparate können vorzugsweise in einer einheitlichen Darreichungsform vorliegen, in der die jeweilige Menge an EPO und Eisenpräparat nebeneinander in einem Behältnis vorliegen.

Im Sinne der Erfindung kommen als Eisenpräparate orale oder parenterale Darreichungsformen in Frage. Hierbei kann es sich grundsätzlich um Einzelpräparate handeln, die als Wirkstoff ein physiologisch verträgliches Eisensalz oder eine Eisenkomplex-Verbindung enthalten, oder auch um Kombinationspräparate, die neben dem physiologisch verträglichen Eisenpräparat weitere Wirkstoffe, wie z.B. Vitamine. Folsäure, Thiaminchlorid, Riboflavin, Pyridoxin, Ascorbinsäure, Nicotinamid, Calciumpantothenat, etc., enthalten.

Physiologisch verträgliche Eisensalze oder Eisenkomplex-Verbindungen sind beispielsweise Eisen(II)-sulfat, Eisen(II)-fumarat, Eisen(III)-citrat, Eisen(II)-gluconat, Eisen(II)-succinat, Eisen(II)-chlorid, Eisen(II)-glycin-sulfat-Komplex. Eisen(II)-aspartat, Natrium-Eisen(III)-gluconat-Komplex. Eisen(III)-hydroxid-Polymaltose-Komplex oder Ferri-Sorbitol-Zitrat-Komplex. Bevorzugte Eisen-präparate sind insbesondere Fe(III)-Komplexe, insbesondere solche mit einem Molekulargewicht zwischen 30.000 und 100.000 D. Besonders bevorzugt ist Fe(III)-Saccharat. Hier kann auf das kommerziell erhältliche Präparat "Ferrum Vitis" (Fa. Neopharma, Deutschland) zurückgegriffen werden. Durch die erfindungsgemäße geringe Eisendosierung ist es auch möglich, labile Eisen-Komplexe, wie das Eisen-Gluconat (MG ca. 1000 D; Ferrlecit), im Kombinationspräparat einzusetzen, obwohl diese labilen Eisenkomplexe relativ große Mengen ionisiertes Eisen freisetzen, was bei der intravenösen Applikation größerer Mengen zu Toxizitäten führen würde.

Im folgenden wird bei Bezugnahme auf die Menge des Eisenpräparates grundsätzlich die zu applizierende äquivalente Menge an Eisenionen, Fe(II)- oder Fe(III)-ionen, verstanden. Durch diese Standardisierung kann die Menge eines beliebigen Eisenpräparates auf Basis dessen bekannten Molekulargewichts berechnet werden. Im Fall des Eisen-(III)-gluconat x 2 $H_2O$ beträgt beispielsweise die Menge an Eisen 80,5 mg, wenn eine Menge von 695 mg des Eisenpräparates verabreicht wird. Bei Verabreichung von beispielsweise 280 mg wasserfreiem Eisen(II)-succinat beträgt die Eisenmenge 95.2 mg.

Anstelle des Proteins rhEPO (vgl. Europäische Patentschrift EP 0,205,564; EP 0,411,678) können auch Modifikationen des Proteins mit höherem oder geringerem Molekulargewicht als 34.000 Da (Molekulargewicht des urinären EPO), Isoformen des Enzyms oder Proteine mit unterschiedlicher Glykosylierung verwendet werden. Ferner kommen grundsätzlich auch solche Proteine in Frage, die sich durch Deletionen. Substitutionen oder Verlängerungen von einzelnen oder mehreren Aminosäuren von der Aminosäuresequenz des natürlichen EPO mit einer Länge von 166 Aminosäuren ableiten.

Derartige Proteine besitzen im wesentlichen vergleichbare physiologische Eigenschaften wie rhEPO. Insbesondere weisen derartige Proteine biologische Eigenschaften auf, daß Knochenmarkszellen veranlasst werden, die Produktion von Retikulozyten und roten Blutkörperchen zu steigern und/oder die Hämoglobinsynthese oder Eisenaufnahme zu steigern. Anstelle derartiger Proteine können auch niedermolekulare Substanzen verwendet werden, die als EPO-Mimetika bezeichnet werden, und die an den gleichen biologischen Rezeptor binden. Diese Mimetika können vorzugsweise auch oral verabreicht werden. Die zu verabreichende Menge derartiger Proteine oder Mimetika wird ermittelt durch Vergleich der biologischen Aktivitäten zwischen EPO und diesen Wirkstoffen.

Für die Behandlung von Hämodialysepatienten beinhaltet das erfindungsgemäße Kombinationspräparat insbesondere 3.000 bis 7.000 U rhEPO, insbesondere 4.000 bis 6.000 U rhEPO, und vorzugsweise etwa 5.000 U rhEPO. Die Menge an Eisenionen beträgt insbesondere 3 - 20 mg, vorzugsweise 5 - 20 mg und besonders bevorzugt etwa 10 mg. Für die Behandlung von Anämiepatienten beträgt die optimale Dosis 2.000 bis 4.000 U rhEPO, vorzugsweise etwa 3.000 U. Die Menge an Eisenionen beträgt in diesem Fall 3 - 15 mg, insbesondere etwa 5 mg.

Die erfindungsgemäßen Konzentrationen an rhEPO und Eisenkomplex erlauben in ihrer Kombination eine optimale Einstellung und Behandlung von Hämodialyse- oder Anämiepatienten und führen bei intravenöser Eisentherapie nicht zu Akute-Phase-Reaktionen.

Die Behandlung mit dem Kombinationspräparat erfolgt ein- bis fünfmal, bevorzugt bis zu viermal wöchentlich, wobei die Gesamtmenge pro Patient von 60 mg Eisenionen pro Woche im Fall der Behandlung von Hämodialysepatienten nicht überschritten werden sollte. Bei der Behandlung der Anämie sollte die Gesamtmenge von 20 mg pro Woche an Eisenionen nicht überschritten werden. Ein besonderer Vorteil des erfindungsgemäßen Kombinationspräparates in der klinischen Praxis liegt darin, daß es sowohl in der Korrektur- als auch in der Erhaltungsphase der Eisentherapie bei Hämodialysepatienten angewendet werden kann, ohne Toxizitäten hervorzurufen. Bisher wurden unterschiedliche Mengen an Eisen verabreicht, wobei in der Korrekturphase zunächst geringere Dosierungen an Eisenionen im Vergleich zur Erhaltungsphase verabreicht wurden. Überraschenderweise ist diese unterschiedliche Dosierung bei Verwendung der erfindungsgemäßen Kombinationspräparaten nicht mehr erforderlich. Die Menge an EPO und Eisenpräparat sind derart optimal aufeinander abgestimmt, daß eine Unterscheidung zwischen Erhaltungsdosis und Korrekturdosis nicht erforderlich ist. Hierdurch wird eine erhöhte Sicherheit bei der Behandlung von Hämodialyse- oder Anämiepatienten erzielt, da Verwechslungsmöglichkeiten bezüglich der optimalen Dosierung des Eisenpräparates nicht mehr bestehen.

Bei der Anwendung der Kombinationspräparate ist es möglich, rhEPO und Eisenkomplex in einer sogenannten fixen Kombination. d.h. in einer einzigen pharmazeutischen Formulierung zu verabreichen, in der beide Verbindungen enthalten sind. Dies kann z.B. eine Injektionslösung- bzw. Infusionslösung oder deren Lyophilisat sein, die beispielsweise in Ampullen abgefüllt sind. Diese Darreichungsform hat den Vorteil, daß das EPO bei der Herstellung und Lagerung der Darreichungsform durch den Eisenkomplex stabilisiert wird. Im Falle eines Lyophilisates wird nach dessen Auflösen das EPO durch den Eisenkomplex aktiviert. Die fixe Kombination der beiden Wirkstoffe in Form eines Lyophilisates hat den weiteren Vorteil der einfachen und sicheren Handhabung. Das Lyophilisat wird in der Ampulle durch Zugabe pharmazeutisch üblicher Injektionsmedien gelöst und intravenös appliziert.

Es ist auch möglich, EPO und Eisenkomplex in Form von getrennten pharmazeutischen Formulierungen zur Verfügung zu stellen. In der Regel erfolgt dies in Form einer einzigen Verpackungseinheit, die zwei Behältnisse umfaßt, wobei das erste eine geeignete Darreichungsform für EPO ist (Lyophilisat, Injektions- oder Infusionslösung), und das zweite eine geeignete Darreichungsform für das Eisenpräparat darstellt. Diese freie Kombination, die in einer einzigen Verpackungseinheit (Arzneimittelpackung) zur Verfügung gestellt werden kann, hat den Vorteil, daß jedem zu behandelnden Patienten individuell eine direkt zuzuordnende Menge an EPO und Eisenpräparat zugewiesen werden kann. Derartige Kombinationspräparate bieten außerdem den Vorteil der größeren Sicherheit für den Therapieerfolg, da jeweils die optimal abgestimmte Menge der Einzelpräparate festgelegt ist, und eine Verwechslung mit sonst im Handel erhältlichen Einzelpräparten, die in unterschiedlichen Dosierungen angeboten werden, weitgehend ausgeschlossen werden kann. Zudem ist zu berücksichtigen, daß in verschiedenen Ländern oft Arzneimittelpräparate mit unterschiedlichen Dosierungen aufgrund der nationalen Erfordernisse im Handel sind, und somit eine erhöhte Verwechslungsgefahr mit variierenden Mengenverhältnisse der Einzelwirkstoffe (EPO und Eisenkomplex) besteht. Die erfindungsgemäßen Kombinationspräparate minimieren ferner das Risiko einer versehentlich zu hohen Eisengabe, die möglicherweise erfolgen kann, wenn herkömmliche Eisenpräparate aus separaten Arzneimittelpackungen zusammen mit der EPO-Gabe eingesetzt werden. Durch die erfindungsgemäßen Kombinationspräparate wird eine sichere Therpie und einfache Handhabung durch das behandelnde Personal oder im Rahmen der durch den Patienten vorgenommenen Selbstmedikation sichergestellt. Im vorliegenden Fall ist es z.B. auch möglich, einen Wirkstoff als Injektionslösung und den anderen Wirkstoff (Eisenkomplex) als Darreichungsform zur oralen Verabreichung zur Verfügung zu stellen.

Für den Fall, daß der Wirkstoff EPO als Lyophilisat zur Verfügung gestellt wird, enthalten die Arzneimittelpackungen (Kombinationspackungen) die entsprechende Menge an EPO in Glasampullen oder in Karpulen. Das Eisenpräparat kann in fester Form (Tablette, Pulver, Granulat, Lyophilisat, etc.) oder auch in flüssiger Form in einem getrennten Behältnis vorliegen. Ferner enthält die Kombinationspackung vorzugsweise eine Rekonstitutionslösung, um entweder das Wirkstofflyophilisat allein oder auch zusammen mit dem festen Eisenpräparat aufzulösen. Liegt das Eisenpräparat als gebrauchsfertige Lösung vor, kann die Lösung zusammen mit der EPO-Lösung gemischt werden, falls die gemeinsame Applikation von EPO und Eisenpräparat erfolgen soll. Grundsätzlich kann das Eisenpräparat auch als Konzentrat für den Zusatz zu herkömmlichen Infusionslösungen zur Verfügung gestellt werden, wodurch eine langsamere Appliaktion über meherere Stunden hinweg erfolgen kann. In diesem Fall wird ein geringes Volumen der Eisenkomplex-haltigen Lösung (ca. 0,5 - 10 ml) zur gebrauchsfertigen Injektionslösung von ca. 500 - 1000 ml hinzugefügt.

Eine weitere Möglichkeit im Sinne der vorliegenden Erfindung besteht darin, jeweils Einzelpräparate von EPO und Eisenpräparaten als unabhängige Arzneimittel zur Verfügung zu stellen, wobei die Einzelpräparate derart konfektioniert sind, daß sie die erforderlichen Menge der Einzelsubstanzen für die erfindungsgemäße Kombination von EPO und Eisenkomplex enthalten. In der Regel enthalten die Arzneimittelpackungen die vorgeschriebenen Beipackzettel, in denen ein entsprechender Hinweis für die kombinierte Gabe mit EPO bzw. mit Eisenpräparaten in der erforderlichen Menge enthalten ist. Ein entsprechender Hinweis kann auch als Verpackungsaufdruck auf der Arzneimittelpackung (Sekundärpackmittel) oder dem Primärpackmittel (Ampulle. Blisterstreifen, etc.) enthalten sein. So wird im Falle des EPO-haltigen Arzneimittels mit 2.000 - 7.000 Units EPO beispielsweise darauf hingewiesen, daß dieses Präparat insbesondere zusammen mit einem Eisenkomplex-Präparat enthaltend 1 - 20 mg Eisen verabreicht werden sollte. Im Falle der Eisenpräparate wird umgekehrt auf die kombinierte Gabe mit 2000 - 7000 U EPO hingewiesen.

Die Herstellung der pharmazeutischen Darreichungsformen erfolgt nach üblichen, in der galenischen Technik bekannten Verfahren mit pharmazeutisch üblichen Hilfsstoffen.

Bei der Durchführung der Kombinationstherapie mit dem erfindungsgemäßen Kombinationspräparat kann auf sehr einfache Art und Weise über die wöchentliche maximale Dosierung entschieden werden, indem die diagnostischen Parameter für den Eisenstatus, insbesondere die Parameter Eisen, Transferrin, Transferrinsättigung und Ferritin, bestimmt werden. Es zeigte sich, daß der Patient in Korrektur- und Erhaltungsphase optimal eingestellt ist, wenn

Ferritin: 100 - 300 µg/l (entspricht Speicher-Eisen(III) von 800 - 1200 mg), und die
Transferrinsättigung: 20 - 40 %

betragen. Bevorzugt beträgt die Ferritin-Konzentration mindestens 125 µg/l, insbesondere mindestens 150 µg/l, und maximal bis zu 270 µg/l, insbesondere maximal bis zu 250 µg/l. Die Eisenkonzentration beträgt vorteilhaft zwischen 10 - 20 µmol/l (entspricht etwa 56 - 112 µg/dl) und die Transferrinkonzentration zwischen 30 - 60 µmol/l (entspricht etwa 240 - 480 mg/dl). Die Transferrinsättigung ist definiert als das Verhältnis von Serum/Plasma-Eisenkonzentration zu Serum/Plasma-Transferrin-Konzentration (multipliziert mit einem Korrekturfaktor von 1,41). Es handelt sich hierbei um eine dimensionslose Zahl, die unabhängig von Hydratationsstatus des Patienten ist. Die Transferrinsättigung berechnet sich nach der Formel:

$$\text{Transferrinsättigung (\%)} = (\text{Eisen [µg/dl]} \times 100) / (\text{Transferrin [mg/dl]} \times 1{,}41)$$

Eine optimale Einstellung des Patienten ist erreicht, wenn das Verhältnis von Transferrinsättigung (in %) zur Ferritinkonzentration (in µg/l) im Bereich von 5 - 40 % liegt. Dieser Parameter wird definiert als Transferrin/Ferritin-Sättigung (TfF-Sättigung). Er berechnet sich nach der Formel

$$\text{TfF-Sättigung} = (\text{Transferin-Sättigung in \%}) \times 100 / (\text{Ferritin [µg/l]})$$

Bevorzugt liegt der Wert für diesen Parameter im Bereich von 10 - 40, insbesondere bei 15 - 25 [% x 1/ µg].

Mittels dieser Parameter wird z.B. bei Verabreichung von 1 bis 6 Ampullen, vorzugsweise bis zu 4 oder 5 Ampullen, in der Woche (eine Ampulle enthält 2000 - 7000 U rhEPO und 1 - 20 mg Eisenkomplex) die optimale Einstellung des Patienten diagnostisch überprüft.

Um unerwünschte Nebenwirkungen sicher auszuschließen, wird der Akute-Phase-Parameter CRP (5 mg/l ± 100%) [CRP = C-reaktives Protein] gemessen, wobei das CRP zur Zeit als der beste Protein-Marker einer Entzündungsreaktion gilt. Zusätzlich können die Leberparameter GPT (Glutamat-Pyruvat-Transaminase), GOT (Glutamat-Oxalacetat-Transaminase) und γ-GT (Gamma-Glutamyltransferase) bestimmt werdem, die in folgenden Bereichen liegen sollten (Bestimmung bei 37°C): GPT: < 50 U/l; GOT: < 50 U/l; γ-GT: < 40 U/l. Der Parameter GPT steht hierbei gegenwärtig in der Leberdiagnostik an erster Stelle.

Desweiteren können gegebenenfalls die hämatologischen Kontrollparameter wie Hämatokrit (Anteil der roten Blutkörperchen am Gesamtvolumen) oder der Anstieg der hypochromen Erythrocyten herangezogen werden. Zeigen die Kontrollparameter höhere Anstiege, ist die wöchentliche Eisen-Gabe zu reduzieren, dann sollte zusätzlich rhEPO verabreicht werden. Zeigen die Kontrollparameter, vor allem die Transferrinsättigung, geringere Werte, ist die wöchentliche Eisen-Gabe zu erhöhen.

Weiterhin wurde im Sinne der vorliegenden Erfindung überraschenderweise festgestellt, daß die Festlegung einer für den Patienten individuellen, optimalen Therapiedosis von EPO und von Eisenionen zur Behandlung der Anämie durch Bestimmung des löslichen TfR (Transferrinrezeptors) erfolgen kann. Die optimale Therapiedosis von EPO und von Eisen(III) ist dann erreicht, wenn die Konzentration des löslichen TfR nicht mehr ansteigt. Um sicherzugehen, daß genügend mobilisierbares Eisen vorhanden ist, wird die i.v.-Eisen-Dosis und die EPO-Gabe abwechselnd erhöht, bis ein Plateau erreicht ist. Dies entspricht einer Konzentration von 1.500-2.000 μg/l TfR.

Bei der Durchführung der Kombinationstherapie mit dem erfindungsgemäßen Kombinationspräparat zur Behandlung der Anämie kann auf sehr einfache Art und Weise über die wöchentliche maximale Dosierung entschieden werden, indem die diagnostischen Parameter Transferrin-Rezeptor (TfR), Ferritin und das Verhältnis TfR zu Ferritin bestimmt werden. Es zeigte sich, daß der Patient in Korrektur- und Erhaltungsphase optimal eingestellt ist, wenn

Ferritin: 100 - 300 μg/l (entspricht Speicher-Eisen(III) von 400 - 1200 mg)
TfR/Ferritin: > 15

betragen. Die TfR-Konzentration beträgt vorteilhaft zwischen 1500 - 2500 μg/l. Das Verhältnis der Konzentrationen TfR (in μg/l) zu Ferritin (in μg/l) liegt insbesondere im Bereich von 15 - 35, vorzugsweise bei Werten oberhalb von 20.

Mittels dieser Parameter wird z.B. bei Verabreichung von 1 bis 6 Ampullen, vorzugsweise bis zu 4 oder 5 Ampullen, in der Woche (eine Ampulle enthält 3000 U rhEPO und 5 mg Eisenkomplex) die optimale Einstellung der Patienten diagnostisch überprüft. Hierbei handelt es sich insbesondere nicht um Hämodialys-Patienten, sondern um solche Patienten, die aufgrund einer anderweitig bedingten Anämie mit EPO und/oder Eisenpräparaten therapiert werden.

Um unerwünschte Nebenwirkungen sicher auszuschließen, wird der Akute-Phase-Parameter CRP (2 - 10 mg/l) [CRP = C-reaktives Protein] gemessen. Zusätzlich kann der Leberparameter GPT (Glutamat-Pyruvat-Transaminase) bestimmt werden, der < 50 u/l bei 37°C (< 30 u/l bei 25°C) sein sollte. Desweiteren können gegebenenfalls die hämatologischen Kontrollparameter wie Hämatokrit (Anteil der roten Blutkörperchen am Gesamtvolumen) oder der Anstieg der hypochromen Erythrocyten herangezogen werden. Hierbei können die Retikulozyten auf einen Wert von bis zu 15/1000 - 30/1000 steigen. Die typtische Hämoglobin-Konzentration liegt bei 12 -18 g/dl. Zeigt der lösliche TfR einen höheren Anstieg, ist die wöchentliche Eisen-Gabe zu erhöhen auf bis zu 35 mg. Zeigt der lösliche TfR geringere Werte, ist die wöchentliche EPO-Dosis zu erhöhen.

Die Bestimmung des Eisenstatus erfolgt durch Analyse von Proben aus Körperflüssigkeiten (Blut, Serum, Urin etc.) der betreffenden Patienten. Für die Bestimmung des Eisenstatus werden insbesondere die Konzentration von Eisen, Transferrin, Ferritin. Transferrinrezeptor, die Transferrinsättigung und die Transferrin/Ferritin-Sättigung bestimmt. Im Falle von Hämodialysepatienten werden vorzugsweise die Parameter Eisen, Transferrin, Ferritin und Transferrinsättigung nach an sich üblichen Analysenmethoden bestimmt. Relevant ist insbesondere die Bestimmung des Transferrin/Ferritin-Sättigungswertes. Im Falle von Anämie-Patienten, deren Anämie nicht durch Hämodialyse verursacht ist, werden vor allem die Ferritinkonzentration und die Konzentration des Transferrin-Rezeptors bestimmt. Relevant ist insbesondere die Bestimmung des Verhältnisses von Transferrin-Rezeptor zu Ferritin (Transferrin-Rezeptor/Ferritin-Sättigungswert).

Ein in diesem Sinne erfindungsgemäßes optimales Kombinationspräparat für die Behandlung von Anämiepatienten umfaßt 2.000 - 4.000 U EPO und 3 - 10 mg, vozugsweise 5 mg an Eisenionen, vorzugsweise eines Fe(III)-Komplexes, wobei das EPO und der Fe(III)-komplex in getrennten Darreichungsformen oder in einer einheitlichen Darreichungsform vorliegen können.

Die erfindungsgemäßen Darreichungsformen ermöglichen auch eine Applikation der Eisenpräparate 1 bis 3 Tage vor der EPO-Applikation, um die Eisenspeicher bereits vor Beginn der EPO-Behandlung aufzufüllen.

Gegenstand der Erfindung ist auch die Verwendung von 3.000 - 7.000 U rhEPO und 5 - 20 mg an Eisenionen eines physiologisch verträglichen Eisenpräparates zur Herstellung von Kombinationspräparaten zur Behandlung von Hämodialysepatienten.

Zur Untersuchung des Eisenstoffwechsels werden in der klinischen Chemie die Konzentration von Eisen im Blut, und die Eisenbindungskapazität bestimmt. Es sollten immer beide Tests durchgeführt werden, da der Bezug ihrer Meßergebnisse zueinander wichtig ist. Gewöhnlich liegen die normalen Serumeisenspiegel bei Männern zwischen 75 und 150 mg/dl und bei Frauen zwischen 60 und 140 mg/dl. Die totale Eisenbindungskapazität beträgt zwischen 250 und 450 mg/dl. Der Serumeisenspiegel schwankt im Tagesverlauf. Er ist erniedrigt bei Eisenmangel und bei Anämien im Rahmen chronischer Erkrankungen. Er ist erhöht bei Hämolyse und bei Syndromen mit Eisenüberladung (z.B. Hämochromatose oder Hämosiderose). Patienten, die unter einer oralen Eisenmedikation stehen, können normale Eisen-Serumspiegel haben, obwohl eigentlich ein Eisenmangel bei ihnen vorliegt. Die totale Eisenbindungskapazität (= Transferrin x 2) ist erhöht beim Eisenmangel, dagegen aber erniedrigt bei Anämien im Verlauf von chronischen Erkrankungen.

Außerdem wird der Serumferritinspiegel bestimmt. Ferritin ist ein eisenspeicherndes Glykoprotein, von dem gewebetypische Isoferritine existieren und das im Serum immunologisch bestimmt werden kann, z.B. durch einen Radioimmunoassay (RIA) oder auch durch turbidimetrische Methoden. Der Ferritinwert ist ein Maß für die Eisenspeicherung im Gewebe. In den meisten Laboratorien liegt der Normalbereich zwischen 30 und 300 ng/ml, und der geometrische Mittelwert beträgt 88 bei Männern und 49 bei Frauen. Die Serum-Ferritinwerte stehen in enger Beziehung zum Eisengesamtvorrat des Körpers. Deshalb findet man erniedrigte Serum-Ferritinspiegel nur beim Eisenmangel. Erhöhte Spiegel findet man bei Eisenüberladung. Ebenfalls erhöhte Serum-Ferritinspiegel findet man bei Leberschäden oder in Assoziation mit manchen Neoplasien, wo Ferritine auch an Akute-Phase-Proteine gebunden sein können. Auch der Serumtransferrin-Rezeptor kann durch einen enzymverstärkten Immunabsorptionstest (enzyme-linked immunosorbent assay = ELISA) bestimmt werden. Dabei wird ein monoklonaler Antikörper gegen den löslichen Rezeptor verwendet. Der Referenzbereich liegt zwischen 0,5 - 3 mg/l. Der Spiegel ist erhöht bei geringem Mangel in den Eisenspeichern. Die Konzentrationen spezifischer Erythrozyten-Ferritine können bestimmt werden, um die Eisenspeicher zu charakterisieren, besonders dann, wenn das Serum-Ferritin bei Gewebeverletzungen oder durch Akute-Phase-Reaktionen nicht verwertbar ist.

Zur Untersuchung des Eisenstoffwechsels wird ferner auch der Erythrozyten-Ferritin-Spiegel bestimmt. In heparinisiertem Blut werden die Erythrozyten von den Leukozyten und Thrombozyten (die ebenfalls Ferritin enthalten) mittels Zentrifugation getrennt. Es folgen die Lyse der Erythrozyten und die immunologische Bestimmung des gespeicherten Ferritins. Das Erythrozyten-Ferritin spiegelt den Status der Eisenspeicher während der zurückliegenden 3 Monate wider (d.h. während der Lebenszeit eines Erythrozyten). Die Normalwerte liegen im allgemeinen zwischen 5 und 48 Attogramm (ag) pro Erythrozyt. Werte < 5 findet man bei Eisenmangelanämien, erhöhte Werte (oft > 100) bei Eisenüberladung (z.B. Hämochromatose). Ähnliche Aussagekraft hat die Bestimmung von Zinkprotoporphyrin.

Die Erfindung wird anhand der folgenden Beispiele näher erläutert, wobei im Falle der Eisenpräparte sich die Mengenangaben auf die äquivalente Menge an Eisenionen (und nicht auf die Menge des verabreichten Eisenkomplexes) beziehen:

Beispiel 1:    Patient A (Hämodialyse)

a) Standardpräparat

Nach der herkömmlichen Therapiemethode wurden dem Patienten einmal pro Woche 100 mg Eisen(III)-komplex und dreimal pro Woche 5000 U rEPO i.V. verabreicht. Dabei lagen die diagnostischen Parameter Transferrin, Transferrin-Sättigung, CRP, GOT/GPT und $\gamma$-GT im Normalbereich, der Ferritin-Wert erwies sich mit 800 - 1300 $\mu$g/l als zu hoch. Zur Absenkung des Ferritin-Wertes auf < 500 $\mu$g/l wurden dreimal/Woche je 5000 U rEPO über einen Zeitraum von 3 Wochen verabreicht.

b) Erfindungsgemäßes Präparat

Bei nachfolgender dreimaliger Verabreichung/Woche des erfindungsgemäßen Kombinationspräparates bestehend aus 10 mg Eisen(III)-Saccharat und 5000 U rEPO konnte ein Ferritin-Wert im Normalbereich erreicht und bei der weiteren Behandlung gehalten werden. Auch alle anderen Parameter lagen im Normalbereich.

Beispiel 2    Patientin B (Hämodialyse)

a) Standardpräparat

Patientin B erhielt gemäß Beispiel 1 einmal pro Woche 50 mg Eisen(III)-präparat und dreimal pro Woche 5000 U rEPO. Trotz der geringeren Eisendosis als im Beispiel 1 liegen die Parameter Ferritin und Transferrinsättigung zu hoch.

Der Ferritin-Wert wurde auf < 500 $\mu$g/l und die Trasferrinsättigung < 25% durch dreimalige, wöchentliche Gabe von je 5000 U rEPO über einen Zeitraum von 3 Wochen abgesenkt.

b) Erfindungsgemäßes Präparat

Nach zwei- bis dreimaliger Verabreichung des erfindungsgemäßen Kombinationspräparates/Woche von 10 mg Eisen(III)-präparat und 5000 U rEPO lagen alle Werte im Plausibilitätsbereich und es traten auch bei der weiteren Behandlung mit dem erfindungsgemäßen Präparat keine extremen Werte mehr auf.

Beispiel 3      Patient C (Hämodialyse)

a) Standardpräparat

Patient C erhielt zweimal pro Woche 50 mg Eisen(III)-präparat und zweimal pro Woche 2000 U rEPO. Es zeigte sich, daß in diesem Fall der Ferritin-Wert mit 1500 - 2500 µg/l sehr hoch liegt und die γ-GT ansteigend ist. Durch Weglassen der Eisen-Infusionen wurden die Ferritin-Werte auf 500 µg/l innerhalb von 3 Wochen gesenkt.

b) Erfindungsgemäßes Präparat

Wiederum wurden durch nachfolgende dreimalige Verabreichung des erfindungsgemäßen Kombinationspräparates aus 10 mg Eisen(III)-Gluconat und 5000 U rEPO die Normalwerte von Ferritin, Transferrin-Sättigung, CRP, GOT und γ-GT erreicht und bei der weiteren Behandlung gehalten.

Beispiel 4:      Patient D (Anämie-Patient)

a) Standardpräparat

Nach der herkömmlichen Therapiemethode wurden dem Patienten einmal pro Woche 100 mg Eisen(III)-komplex und dreimal pro Woche 5000 U rEPO i.V. verabreicht. Dabei lagen die diagnostischen Parameter Transferrin, Transferrin-Sättigung, CRP, GOT/GPT und γ-GT im Normalbereich, der Ferritin-Wert erwies sich mit 800 - 1300 µg/l als zu hoch, der Wert für den Transferrin-Rezeptor lag zwischen 100 - 500 µg/l, und damit zu niedrig.
Zur Anhebung des Transferrin-Rezeptor-Wertes auf Werte oberhalb von 1500 µg/l und zur Absenkung des Ferritin-Wertes auf Werte unterhalb von 500 µg/l wurden dreimal/Woche je 5000 U rEPO über einen Zeitraum von 3 Wochen verabreicht.

b) Erfindungsgemäßes Präparat

Bei nachfolgender fünfmaliger Verabreichung/Woche des erfindungsgemäßen Kombinationspräparates bestehend aus 5 mg Eisen(III)-Saccharat und 3000 U rEPO konnte ein Transferrin-Rezeptor-Wert und ein Ferritin-Wert im Normalbereich erreicht und bei der weiteren Behandlung gehalten werden. Auch alle anderen Parameter lagen im Normalbereich.

Beispiel 5      Patient E (Anämie-Patient)

a) Standardpräparat

Patient E erhielt gemäß Beispiel 4 einmal pro Woche 50 mg Eisen(III)-präparat und dreimal pro Woche 5000 U rEPO. Trotz der geringeren Eisendosis als im Beispiel 4 liegen die Parameter Ferritin und Transferrinsättigung zu hoch.
Der Transferrin-Rezeptor-Wert wurde auf Werte oberhalb von 1500 µg/l angehoben, und der Ferritin-Wert wurde auf < 500 µg/l und die Transferrinsättigung < 25% durch dreimalige, wöchentliche Gabe von je 5000 U rEPO über einen Zeitraum von 3 Wochen abgesenkt.

b) Erfindungsgemäßes Präparat

Nach vier- bis fünfmaliger Verabreichung des erfindungsgemäßen Kombinationspräparates/Woche von 5 mg Eisen(III)-präparat und 3000 U rEPO lagen alle Werte im Plausibilitätsbereich und es traten auch bei der weiteren Behandlung mit dem erfindungsgemäßen Präparat keine extremen Werte mehr auf.

Beispiel 6      Patient F (Anämie-Patient)

a) Standardpräparat

Patient F erhielt zweimal pro Woche 50 mg Eisen(III)-präparat und zweimal pro Woche 2000 U rEPO. Es zeigte sich. daß in diesem Fall der Transferrin-Rezeptor-Wert mit 100 - 800 µg/l sehr niedrig und der Ferritin-Wert mit 1500 - 2500 µg/l sehr hoch liegt und die γ-GT ansteigend ist. Durch Weglassen der Eisen-Infusionen wurden die Transferrin-Rezeptor-Werte auf Werte oberhalb von 1500 µg/l angehoben und die Ferritin-Werte auf< 500 µg/l innerhalb von 3 Wochen gesenkt.

b) Erfindungsgemäßes Präparat

Wiederum wurden durch nachfolgende fünfmalige Verabreichung des erfindungsgemäßen Kombinationspräparates aus 5 mg Eisen(III)-Gluconat und 3000 U rEPO die Normalwerte von Ferritin, Transferrin-Rezeptor, Transferrin-Sättigung, CRP, GOT und $\gamma$-GT erreicht und bei der weiteren Behandlung gehalten.

**Patentansprüche**

1. Verwendung von 2.000 - 7.000 U EPO und 1 - 20 mg einer äquivalenten Menge an Eisenionen eines physiologisch verträglichen Eisenpräparates zur Herstellung eines pharmazeutischen Kombinationspräparates zur Behandlung von Hämodialysepatienten oder zur Behandlung von Anämien, wobei das Kombinationspräparat sowohl in der Korrekturphase als auch in der Erhaltungsphase der Eisentherapie eingesetzt wird.

2. Verwendung nach Anspruch 1, wobei das Kombinationspräparat ausgewählt ist aus der Gruppe der Präparate enthaltend die folgenden Mengen an EPO und Eisenionen:

   a) 3.000 - 7.000 U EPO und 5 - 20 mg Eisenionen;
   b) 5.000 U EPO und 10 mg Eisenionen;
   c) 2.000 - 4.000 U EPO und 3 - 10 mg Eisenionen; oder
   d) 3.000 U EPO und 5 mg Eisenionen.

3. Verwendung nach Anspruch 1 oder 2, wobei das Kombinationspräparat zur Behandlung von Hämodialysepatienten oder zur Behandlung von Anämien eingesetzt wird.

4. Verwendung von EPO zur Herstellung von Kombinationspräparaten enthaltend 2.000 - 7.000 U EPO zur kombinierten Gabe zusammen mit 1 - 20 mg einer äquivalenten Menge an Eisenionen eines physiologisch verträglichen Eisenpräparates zur Anwendung sowohl in der Korrekturphase als auch der Erhaltungsphase während der Eisentherapie bei der Behandlung von Hämodialysepatienten.

5. Verwendung von EPO zur Herstellung von Kombinationspräparaten enthaltend 2.000 - 4.000 U EPO zur kombinierten Gabe zusammen mit 3 - 10 mg einer äquivalenten Menge an Eisenionen eines physiologisch verträglichen Eisenpräparates zur Anwendung sowohl in der Korrekturphase als auch der Erhaltungsphase während der Eisentherapie bei der Behandlung von Anämien.

6. Pharmazeutische Verpackungseinheit umfassend 2.000 - 7.000 U EPO und 1 bis 20 mg einer äquivalenten Menge an Eisenionen eines physiologisch verträglichen Eisenpräparates als einheitliche Darreichungsform in einem Behältnis oder als getrennte Darreichungsformen in getrennten Behältnissen zur Anwendung sowohl in der Korrekturphase als auch in der Erhaltungsphase während der Eisentherapie.

7. Verpackungseinheit nach Anspruch 6, **dadurch gekennzeichnet, daß** jeweils das EPO und das Eisenpräparat in getrennten Darreichungsformen als Injektions- bzw. Infusionslösungen oder als Lyophilisate vorliegen.

8. Verfahren zur Bestimmung des Eisenstatus in einer Probe aus Körperflüssigkeiten, wobei in einem ersten Schritt die Konzentrationen von Eisen, Transferrin und Ferritin sowie die Transferrinsättigung und in einem zweiten Schritt die Transferrin/Ferritinsättigung bestimmt werden.

9. Verfahren nach Anspruch 8 zur Bestimmung des Eisenstatus bei Hämodialyse-Patienten.

10. Verfahren zur Bestimmung des Eisenstatus in einer Probe aus Körperflüssigkeiten, wobei in einem ersten Schritt die Konzentrationen von Ferritin und des Transferrin-Rezeptors (TfR) und in einem zweiten Schritt die Transferrin-Rezeptor/Ferritin-Sättigung bestimmt werden.

11. Verfahren nach Anspruch 10 zur Bestimmung des Eisenstatus bei Anämie-Patienten.

**Claims**

1. Use of 2,000-7,000 U of EPO and 1-20 mg of an equivalent amount of iron ions of a physiologically compatible iron preparation for the production of a pharmaceutical combination preparation for the treatment of haemodialysis patients or for the treatment of anaemias, with the combination preparation being used not only in the correction phase but also in the maintenance phase of iron therapy.

2. Use according to claim 1, wherein the combination preparation is selected from the group of preparations containing the following amounts of EPO and iron ions:

   a) 3,000-7,000 U of EPO and 5-20 mg of iron ions;
   b) 5,000 U of EPO and 10 mg of iron ions;
   c) 2,000-4,000 U of EPO and 3-10 mg of iron ions; or
   d) 3,000 U of EPO and 5 mg of iron ions.

3. Use according to claim 1 or 2, wherein the combination preparation is used for the treatment of haemodialysis patients or for the treatment of anaemias.

4. Use of EPO for the production of combination preparations containing 2,000-7,000 U of EPO for the combined administration together with 1-20 mg of an equivalent amount of iron ions of a physiologically compatible iron preparation for use not only in the correction phase but also in the maintenance phase during iron therapy in the treatment of haemodialysis patients.

5. Use of EPO for the production of combination preparations containing 2,000-4,000 U of EPO for the combined administration together with 3-10 mg of an equivalent amount of iron ions of a physiologically compatible iron preparation for use not only in the correction phase but also in the maintenance phase during iron therapy in the treatment of anaemias.

6. A pharmaceutical package unit comprising 2,000-7,000 U of EPO and 1 to 20 mg of an equivalent amount of iron ions of a physiologically compatible iron preparation as an integrated administration form in a single container or as separate administration forms in separate containers for use not only in the correction phase but also in the maintenance phase during iron therapy.

7. A package unit according to claim 6, **characterized in that** the EPO and the iron preparation are each present in separate administration forms as injection or infusion solutions or as lyophilizates.

8. A method for the determination of the iron status in a sample of body fluids, wherein the concentrations of iron, transferrin and ferritin as well as the transferrin saturation are determined in a first step and the transferrin/ferritin saturation is determined in a second step.

9. A method according to claim 8 for the determination of the iron status in haemodialysis patients.

10. A method for the determination of the iron status in a sample of body fluids, wherein the concentrations of ferritin and the transferrin receptor (TfR) are determined in a first step and the transferrin receptor/ferritin saturation is determined in a second step.

11. A method according to claim 10 for the determination of the iron status in anaemia patients.

**Revendications**

1. Utilisation de 2000 à 7000 U d'EPO et de 1 à 20 mg d'une quantité équivalente d'ions fer d'une préparation à base de fer tolérée physiologiquement pour la fabrication d'une préparation combinée pharmaceutique destinée au traitement des patients hémodialysés ou au traitement d'anémies, la préparation combinée étant mise en oeuvre aussi bien au cours de la phase de correction qu'au cours de la phase de maintien de la thérapie à base de fer.

2. Utilisation selon la revendication 1, la préparation combinée étant choisie parmi le groupe des préparations contenant les quantités suivantes d'EPO et d'ions fer :

a) 3000 à 7000 U d'EPO et 5 à 20 mg d'ions fer ;
b) 5000 U d'EPO et 10 mg d'ions fer ;
c) 2000 à 4000 U d'EPO et 3 à 10 mg d'ions fer ; ou
d) 3000 U d'EPO et 5 mg d'ions fer.

3. Utilisation selon la revendication 1 ou 2, la préparation combinée étant mise en oeuvre pour le traitement des patients hémodialysés ou pour le traitement d'anémies.

4. Utilisation de l'EPO pour la fabrication de préparations combinées contenant de 2000 à 7000 U d'EPO pour l'administration combinée avec 1 à 20 mg d'une quantité équivalente d'ions fer d'une préparation à base de fer tolérée physiologiquement destinée à être utilisée aussi bien dans la phase de correction que dans la phase de maintien au cours de la thérapie à base de fer dans le traitement des patients hémodialysés.

5. Utilisation de l'EPO pour la fabrication de préparations combinées contenant de 2000 à 4000 U d'EPO pour l'administration combinée avec 3 à 10 mg d'une quantité équivalente d'ions fer d'une préparation à base de fer tolérée physiologiquement destinée à être utilisée aussi bien dans la phase de correction que dans la phase de maintien au cours de la thérapie à base de fer dans le traitement des anémies.

6. Unité de conditionnement pharmaceutique comprenant 2000 à 7000 U d'EPO et 1 à 20 mg d'une quantité équivalente d'ions fer d'une préparation à base de fer tolérée physiologiquement, sous forme de dose unitaire dans un contenant ou sous forme de doses séparées dans des contenants séparés, destinée à être utilisée aussi bien dans la phase de correction que dans la phase de maintien au cours de la thérapie à base de fer.

7. Unité de conditionnement selon la revendication 6, **caractérisée en ce que** respectivement l'EPO et la préparation à base de fer se présentent sous des doses séparées comme solutés prêts à injecter ou à perfuser ou comme lyophylisats.

8. Procédé de détermination du niveau de fer dans un échantillon de fluides corporels, les concentrations en fer, transferrine et ferritine ainsi que la saturation en transferrine étant déterminées dans une première étape, la saturation en transferrine/ferritine étant déterminée dans une deuxième étape.

9. Procédé selon la revendication 8 pour la détermination du niveau de fer chez les patients hémodialysés.

10. Procédé de détermination du niveau de fer dans un échantillon de fluides corporels, les concentrations en ferritine et en récepteur de transferrine (TfR) étant déterminées dans une première étape et la saturation en récepteur de transferrine/ferritine étant déterminée dans une deuxième étape.

11. Procédé selon la revendication 10 pour la détermination du niveau de fer chez les patients au traitement d'anémie.